# FASCICULE DE BREVET EUROPEEN

(11) **EP 0 980 345 B1**
(45) Date de publication et mention de la délivrance du brevet: **11.12.2002**
(21) Numéro de dépôt: 98924264.9
(22) Date de dépôt: 28.04.1998
(51) Int. Cl.: C07C 17/278, C07C 19/08

(54) **PROCEDE DE PREPARATION DE 1,1,1,3,3-PENTACHLOROBUTANE**
NEUE ARZNEIMITTEL AUF DER BASIS VON POLYMEREN AUS MIT METHACRYLAMID MODIFIZIERTER GELATINE
METHOD FOR PREPARING 1,1,1,3,3-PENTACHLOROBUTANE

(30) Priorité: 05.05.1997 BE 9700399; 08.08.1997 BE 9700669; 24.02.1998 BE 9800140
(43) Date de publication de la demande: 23.02.2000
(73) Titulaire: SOLVAY (Société Anonyme), 1050 Bruxelles (BE)
(72) Inventeur: SCHOEBRECHTS, Jean-Paul, B-5980 Grez-Doiceau (BE); MATHIEU, Véronique, B-1300 Wavre (BE); JANSSENS, Francine, B-1800 Vilvoorde (BE)
(74) Mandataire: Jacques, Philippe
(86) Numéro de dépôt international: EP9802585
(87) Numéro de publication internationale: WO98050329

(56) Documents cités:
- EP-A- 0 787 707
- WO-A-97/07083
- US-A- 3 862 978

## Description

La présente invention concerne un procédé de préparation de 1,1,1,3,3-pentachlorobutane, plus particulièrement par télomérisation entre du tétrachlorure de carbone et du 2-chloroprop-1-ène.

Le 1,1,1,3,3-pentachlorobutane présente un intérêt industriel non négligeable dans la mesure où il conduit par fluoration au dérivé pentafluoré correspondant (HFC-365mfc) utilisable notamment comme agent gonflant ou solvant de la troisième génération, dépourvu d'effet néfaste sur la couche d'ozone et ne participant pas au réchauffement global de la planète par effet de serre.

R. FREIDLINA et coll. (Bull. Acad. Sci. USSR, 28, 1766-69, 1979) ont obtenu du 1,1,1,3,3-pentachlorobutane avec un faible rendement par réaction entre le tétrachlorure de carbone et le 2-chloroprop-1-ène en présence de fer pentacarbonyle comme catalyseur, dans l'éthanol ou l'isopropanol. Le faible rendement, d'une part, et la toxicité élevée du catalyseur, d'autre part, font que cette méthode de synthèse apparaît difficilement utilisable industriellement.

Une autre voie d'accès au 1,1,1,3,3-pentachlorobutane, décrite récemment par KOTORA et ses collaborateurs (React. Kinet. Catal. Lett. 44(2), 415-9, 1991), consiste à faire réagir du 1,1,1-trichloroéthane avec du 1,1-dichloroéthène en présence de chlorure cuivreux et d'amine. Le rendement obtenu est faible (8 %) et cette méthode de synthèse apparaît dès lors également difficilement exploitable industriellement.

La demande de brevet EP-787707-1 fait partie de l'état de la technique pour les Etats BE, DE, ES, FR, GB, GR, IT, NL en vertu de l'Article 54(3)/CBE.

L'invention vise dès lors à fournir un procédé qui permet d'accéder, en une seule étape et aux dépens de réactifs facilement accessibles, au 1,1,1,3,3-pentachlorobutane avec d'excellents rendements.

L'invention concerne donc un procédé de fabrication de 1,1,1,3,3-pentachlorobutane par réaction entre du tétrachlorure de carbone et du 2- chloroprop-1-ène en présence d'un catalyseur comprenant au moins un composé de cuivre (I) ou un composé de cuivre (II). En règle générale, les composés du cuivre (Il) sont préférés.

Le composé de cuivre (I) ou (II) est de préférence choisi parmi les halogénures de cuivre, les carboxylates de cuivre, les sels mixtes de cuivre ou les complexes formés avec des ligands neutres.

Parmi les halogénures de cuivre (I) ou (II), on trouve notamment les fluorures, les chlorures, les bromures ou les iodures. Les chlorures et les iodures sont préférés. Le chlorure de cuivre (II) est particulièrement préféré.

Parmi les sels mixtes de cuivre (I) ou (II), on trouve notamment les hydroxyhalogénures. L'hydroxychlorure de cuivre (II) est préféré.

Parmi les carboxylates de cuivre (I) ou (II), on trouve notamment les sels formés au départ d'acides carboxyliques tels que l'acide acétique, l'acide propionique, l'acide butyrique, l'acide cyclohexanebutyrique ou l'acide benzoïque. Les acétates de cuivre (I) ou (II), c'est-à-dire les sels formés aux dépens de l'acide acétique, sont préférés. Le cyclohexanebutyrate de cuivre (II) est tout particulièrement préféré.

Parmi les complexes formés avec des composés du cuivre (I) ou (II), on trouve notamment les complexes formés avec des ligands neutres tels que les phosphines comme la triphénylphosphine ou avec l'acétylacétone. L'acétylacétonate de cuivre (II) est préféré.

Avantageusement, le catalyseur est choisi parmi l'acétate de cuivre (I) ou (II), le cyclohexanebutyrate de cuivre (II), le complexe formé entre le chlorure cuivreux et la triphénylphosphine, l'acétylacétonate de cuivre (II), l'hydroxychlorure de cuivre (II), le chlorure de cuivre (I), l'iodure de cuivre (I) ou le chlorure de cuivre (II). Parmi ces catalyseurs, le chlorure de cuivre (II), l'acétate de cuivre (II), l'hydroxychlorure de cuivre (II), le cyclohexanebutyrate de cuivre (II) et l'acétylacétonate de cuivre (II) sont préférés.

Dans un procédé en discontinu, le rapport molaire entre le composé de cuivre engagé et le 2-chloroprop-1-ène est en général supérieur ou égal à 0,001. Avantageusement, il est supérieur ou égal à 0,002. De préférence, il est supérieur ou égal à 0,005. Le rapport molaire entre le composé de cuivre engagé et le 2 chloroprop-1-ène est le plus souvent inférieur ou égal à 5. Avantageusement, il est inférieur ou égal à 1. De préférence, il est inférieur ou égal à 0,5. D'une manière particulièrement préférée, ce rapport est supérieur ou égal à 0,01 et inférieur ou égal à 0,1.

Dans un procédé en continu, le rapport molaire entre le catalyseur engagé et le 2-chloroprop-1-ène évolue approximativement entre les mêmes limites que dans un procédé en discontinu mais il peut toutefois atteindre la valeur de 50.

Il est entendu que la quantité de catalyseur mise en oeuvre est exprimée, dans un procédé en discontinu, par rapport à la quantité initiale de 2-chloroprop-1-éne mise en oeuvre et, dans un procédé en continu, par rapport à la quantité stationnaire de 2-chloroprop-1-ène présent dans le réacteur.

Le rapport molaire entre le tétrachlorure de carbone et le 2-chloroprop-1-ène mis en oeuvre peut varier dans de larges mesures. Ce rapport est en général égal ou supérieur à 0,1. Avantageusement, ce rapport est égal ou supérieur à 0,5. D'une manière préférée, il est supérieur ou égal à 1. Généralement, ce rapport est toutefois égal ou inférieur à 20. Avantageusement, ce rapport est égal ou inférieur à 10. D'une manière préférée, ce rapport est égal ou inférieur à 8.

Classiquement, la réaction se déroule à une température supérieure ou égale à la température ambiante. De préférence, la température est égale ou supérieure à 40 °C. Avantageusement, elle est égale ou supérieure à 80 °C. En général, la température de réaction ne dépasse pas 200 °C. Avantageusement, notamment avec de l'hydroxychlorure de cuivre (II) comme catalyseur, la température de réaction est supérieure ou égale à 90 °C, de préférence elle est supérieure ou égale à 100 °C. Elle est habituellement inférieure ou égale à 150 °C, plus précisément inférieure ou égale à 140 °C. Avec de l'hydroxychlorure de cuivre (II), il est tout particulièrement avantageux d'effectuer la réaction à une température proche de 130 °C.

La durée de la réaction dans un procédé en discontinu, ou le temps de séjour dans un procédé en continu, sont fonction de divers paramètres tels que la température de réaction, la concentration en réactifs et en catalyseur dans le mélange réactionnel et leurs rapports molaires. En général, en fonction de ces paramètres, le temps de séjour ou la durée de la réaction peuvent varier de 5 secondes à 20 heures.

La pression est habituellement supérieure ou égale à la pression atmosphérique et égale ou inférieure à 15 bars. Elle est avantageusement inférieure ou égale à 10 bars. Comme la réaction de télomérisation est généralement réalisée en phase liquide, la pression est avantageusement choisie, en fonction de la température du milieu réactionnel, de façon à maintenir le milieu réactionnel essentiellement en phase condensée.

Dans un premier mode de mise en oeuvre du procédé selon l'invention, la réaction est réalisée en présence d'un solvant. Tout solvant dans lequel les réactifs forment le produit recherché avec un rendement satisfaisant peut être utilisé. Avantageusement, le solvant de la réaction est un alcool, un nitrile, un amide, une lactone, un oxyde de trialkylphosphine, un phosphate de trialkyle ou un autre solvant polaire.

Parmi les alcools utilisables comme solvant pour la réaction, on trouve notamment le méthanol, l'éthanol, l'isopropanol et le tert-butanol. Parmi les nitriles utilisables comme solvant pour la réaction, on trouve notamment des nitriles aliphatiques, notamment l'acétonitrile, le propionitrile, ou l'adiponitrile et des nitriles aromatiques, notamment le benzonitrile ou le tolunitrile. Parmi les nitriles, le propionitrile et l'adiponitrile sont préférés. Parmi les amides utilisables comme solvant pour la réaction, on trouve des amides linéaires comme le N,N-diméthylacétamide et le N,N-diméthylformamide et des amides cycliques comme la N-méthylpyrrolidone. On peut aussi mentionner l'hexaméthylphosphoramide. Parmi les lactones utilisables comme solvant pour la réaction, on peut citer notamment la γ-butyrolactone. Parmi les oxydes de trialkylphosphine utilisables comme solvant pour la réaction, on peut citer notamment les composés de formule (R1R2R3)PO, dans laquelle R1, R2 et R3 représentent des groupements alkyles en C3-C10, identiques ou différents, de préférence linéaires. On retient en particulier l'oxyde de tri-(n-butyl)phosphine, l'oxyde de tri-(n-hexyl)phosphine, l'oxyde de tri-(n-octyl)phosphine, l'oxyde de n-octyl-di-(n-hexyl)-phosphine et l'oxyde de n-hexyl-di-(n-octyl)-phosphine et leurs mélanges. Parmi les phosphates de trialkyle utilisables comme solvant pour la réaction, on peut citer notamment les composés de formule (RO)₃PO, dans laquelle R représente un groupement alkyle en C3-C10, de préférence linéaire. On retient en particulier le phosphate de tributyle. Comme autres solvants polaires, on peut encore mentionner la 1,3-diméthyl-2-imidazolidinone, le diméthylsulfoxyde et le tétrahydrofuranne. De préférence, le solvant est un amide ou un oxyde de trialkylphosphine. De bons résultats ont en particulier été obtenus avec la N-méthylpyrrolidone, avec le N,N-diméthylacétamide, ainsi qu'avec un mélange d'oxyde de tri-(n-hexyl)phosphine, d'oxyde de tri-(n-octyl)phosphine, d'oxyde de n-octyl-di-(n-hexyl)-phosphine et d'oxyde de n-hexyl-di-(n-octyl)-phosphine.

La quantité de solvant engagé dans le premier mode de mise en oeuvre du procédé selon l'invention n'est pas critique. Toutefois, une solution trop diluée n'est pas favorable à un rendement et à un taux de conversion élevés. De préférence, le rapport molaire du solvant au 2-chloroprop-1-ène est égal ou supérieur à 0,05. Avantageusement, ce rapport est égal ou supérieur à 0,1. Le rapport molaire du solvant au 2-chloroprop-1-ène est en général égal ou inférieur à 20. Avantageusement, il est égal ou inférieur à 15. D'une manière préférée, ce rapport est égal ou supérieur à 0,2 et égal ou inférieur à 10. Dans le milieu réactionnel, la quantité de solvant peut varier, sur une base molaire, d'environ 5 à environ 500 fois la quantité de catalyseur, de préférence d'environ 10 à environ 200 fois.

Dans un deuxième mode, préféré, de mise en oeuvre du procédé selon l'invention, la réaction est réalisée en présence d'un cocatalyseur. Le cocatalyseur peut être choisi notamment parmi les amines, les amides et les oxydes de trialkylphosphine. Comme amines utilisables comme cocatalyseur, on peut citer les amines aliphatiques ou les amines aromatiques. Parmi les amines aliphatiques, on trouve les amines primaires, les amines secondaires et les amines tertiaires. D'une façon générale, on utilise comme amine les alcanolamines, les alkylamines, comme par exemple l'éthanolamine, la n-butylamine, la tert-butylamine, la n-propylamine, l'isopropylamine, la benzylamine, l'hexaméthylène diamine, la diéthylamine, la triéthylamine ou des amines aromatiques comme la pyridine ou l'aniline. Comme amides utilisables comme cocatalyseur, on peut citer la N-méthylpyrrolidone et le N,N-diméthylformamide. Comme oxydes de trialkylphosphine utilisables comme cocatalyseur, on peut citer les mêmes composés que ceux utilisables comme solvant dans le premier mode de réalisation de l'invention. Les alkylamines aliphatiques comme la n-butylamine, la tert-butylamine, la n-propylamine et l'isopropylamine sont des cocatalyseurs préférés. L'isopropylamine et la tert-butylamine sont tout particulièrement préférées. Les oxydes de trialkylphosphine sont d'autres cocatalyseurs préférés.

Les composés de cuivre (II) sont particulièrement préférés lorsqu'on réalise la réaction en présence d'un cocatalyseur. De très bons résultats ont été obtenus avec l'acétate de cuivre (II) comme catalyseur et la tert-butylamine comme cocatalyseur.

Dans ce deuxième mode de mise en oeuvre préféré du procédé selon l'invention, la température de réaction peut être égale ou inférieure à 120 °C. En particulier, la présence du cocatalyseur permet de réaliser la réaction à une température égale ou inférieure à 100 °C tout en conservant un taux de transformation élevé et une excellente sélectivité. Une température proche de 90 °C est tout particulièrement recommandée.

Le rapport molaire entre le cocatalyseur et le 2-chloroprop-1-ène engagé est en général supérieur ou égal à 0,001. Avantageusement, ce rapport est égal ou supérieur à 0,005. Ce rapport est habituellement égal ou inférieur à 1. Avantageusement, ce rapport est égal ou inférieur à 0,5. D'une manière préférée, ce rapport est supérieur ou égal à 0,01 et inférieur ou égal à 0,25. D'une manière particulièrement préférée, ce rapport est supérieur ou égal à 0,1 et inférieur ou égal à 0,2. La quantité de cocatalyseur mise en oeuvre peut varier, sur une base molaire, d'environ 0,1 à environ 25 fois la quantité de catalyseur, de préférence d'environ 0,5 à environ 20 fois.

La présence d'un cocatalyseur dans le mélange réactionnel n'exclut pas l'utilisation d'un nitrile ou d'un autre composé comme solvant.

Dans ce deuxième mode de mise en oeuvre du procédé selon l'invention, le tétrachlorure de carbone peut servir à la fois de réactif et de solvant. Le rapport molaire entre le tétrachlorure de carbone et le 2-chloroprop-1-ène est alors supérieur ou égal à 2. Avantageusement, ce rapport est supérieur ou égal à 4. D'une manière préférée, ce rapport est supérieur ou égal à 4,5. Toutefois, pour éviter une trop grande dilution des réactifs, ce rapport est généralement inférieur ou égal à 10 et de préférence, il est inférieur ou égal à 8. D'une manière préférée, ce rapport est inférieur ou égal à 6.

Le procédé de l'invention permet donc de synthétiser le 1,1,1,3,3-pentachlorobutane en une seule étape, au départ de réactifs facilement accessibles, avec typiquement une sélectivité de plus de 90 %.

Le 1,1,1,3,3-pentachlorobutane obtenu selon le procédé de l'invention est un précurseur de l'analogue pentafluoré 1,1,1,3,3-pentafluorobutane (HFC-365mfc) correspondant, lequel peut être facilement obtenu par traitement avec du fluorure d'hydrogène en présence d'un catalyseur tel qu'un sel d'antimoine, un sel de titane, un sel de tantale ou un sel d'étain.

Les exemples ci-après illustrent l'invention de manière non limitative. Dans ces exemples, on a introduit les réactifs, le solvant et le catalyseur dans un autoclave de 300 ml dont les parois internes sont recouvertes de TEFLON®. Ensuite, l'appareil a été fermé hermétiquement, placé dans un four vertical et la température a été augmentée progressivement et maintenue à la valeur désirée pendant plusieurs heures. L'agitation a été assurée par un barreau magnétique placé dans le fond de l'autoclave. En fin de réaction, on a laissé refroidir l'autoclave, un échantillon de liquide a été prélevé à la seringue et dosé par une méthode chromatographique pour déterminer le taux de conversion du 2-chloroprop-1-ène et la sélectivité en 1,1,1,3,3-pentachlorobutane.

Dans les tableaux ci-dessous, le taux de conversion est le rapport, exprimé en pourcents, entre la quantité de 2-chloroprop-1-ène mise en oeuvre diminuée de la quantité non convertie au terme de la réaction et la quantité mise en oeuvre; la sélectivité en 1,1,1,3,3-pentachlorobutane est le rapport, exprimé en pourcents, entre la quantité de 1,1,1,3,3-pentachlorobutane formé et la quantité de 1,1,1,3,3-pentachlorobutane qui aurait été formée si tout le 2-chloroprop-1-ène converti avait généré du 1,1,1,3,3-pentachlorobutane.

### Exemples 1 à 4

Dans ces exemples, on a préparé du 1,1,1,3,3-pentachlorobutane en présence de différents nitriles et en présence d'un mélange contenant 20 % de CuCl et 80 % de CuClOH (et symbolisé par CuCl-CuClOH ci-dessous) à titre de composé de cuivre. Le rapport molaire 2-chloroprop-1-ène/CCl₄/CuCl-CuClOH/nitrile était 1/2/0,01/1. La réaction s'est déroulée à 130 °C durant 13 heures. Les résultats obtenus sont rassemblés dans le tableau I.

**Tableau I**

| Exemple | Nitrile | Conversion | Sélectivité |
|---|---|---|---|
| 1 | Propionitrile | 94 | 100 |
| 2 | Benzonitrile | 92 | 92 |
| 3 | Tolunitrile | 94 | 91 |
| 4 | Adiponitrile | 97 | 96 |

### Exemple 5

On a répété l'exemple 1 avec un rapport molaire 2-chloroprop-1-ène/nitrile de 1/0,5. On a obtenu un taux de conversion de 85 % et une sélectivité de 85 %.

### Exemple 6

On a répété l'exemple 1 avec un rapport molaire 2-chloroprop-1-ène/nitrile de 1/4. On a obtenu un taux de conversion de 90 % et une sélectivité de 89 %.

### Exemple 7

On a répété l'exemple 6 en utilisant du chlorure de cuivre (II) anhydre comme catalyseur. On a obtenu un taux de conversion de 92 % et une sélectivité de 98 %.

### Exemples 8 et 9

Dans ces exemples, on a répété l'exemple 6 à différents rapports molaires entre le 2-chloroprop-1-ène et le CuCl-CuClOH. Le rapport molaire 2-chloroprop-1-ène/CCl₄/propionitrile était 1/2/4. Les résultats obtenus sont rassemblés dans le tableau II.

**Tableau II**

| Exemple | Rapport molaire CuCl-CuClOH/ 2-chloroprop-1-ène | Conversion | Sélectivité |
|---|---|---|---|
| 8 | 0,10 | 96 | 99 |
| 9 | 0,06 | 98 | 92 |

### Exemple 10

Dans cet exemple, on a répété l'exemple 6 à une température de 150 °C. On a obtenu un taux de conversion de 99 % et une sélectivité de 91 %.

### Exemples 11 et 12

Dans ces exemples, on a préparé du 1,1,1,3,3-pentachlorobutane en présence de différentes amines et en présence de CuCl-CuClOH. Le rapport molaire 2 chloroprop-1-ène/CCl₄/CuCl-CuClOH/amine était 1/5/0,05/0,1. La réaction s'est déroulée à 90 °C durant 2 heures. Les résultats obtenus sont rassemblés dans le tableau III.

**Tableau III**

| Exemple | Amine | Conversion | Sélectivité |
|---|---|---|---|
| 11 | isopropylamine | 95 | 96 |
| 12 | tert-butylamine | 79 | 95 |

### Exemple 13

On a répété l'exemple 12 avec de l'acétate de cuivre (II) comme catalyseur et un temps de réaction de 1 heure à 90 °C. Le rapport molaire 2-chloroprop-1- ène/CCl₄/Cu(COOCH₃)₂/amine était de 1/5/0,05/0,15. On a obtenu un taux de conversion de 96 % et une sélectivité de 97 %.

### Exemples 14 à 20

On a répété l'exemple 11 avec divers composés du cuivre. Les résultats sont rassemblés dans le tableau IV.

**Tableau IV**

| Exemple | Composé de cuivre | Conversion | Sélectivité |
|---|---|---|---|
| 14 | (CH₃COO)₂Cu | 88 | 98 |
| 15 | CuI | 89 | 98 |
| 16 | Cu(PPh₃)₃Cl | 66 | 99 |
| 17 | CuBr | 68 | 98 |
| 18 | (C₁₀H₁₇O₂)₂Cu* | 92 | 98 |
| 19 | (C₅H₇O₂)₂Cu** | 82 | 93 |
| 20 | CuClOH | 87 | 96 |

| | | | |
|---|---|---|---|
| * : cyclohexanebutyrate de cuivre (II) | | | |
| ** : acétylacétonate de cuivre (II) | | | |

### Exemple 21 (non conforme à l'invention)

On a répété l'exemple 6 mais en l'absence de composé de cuivre. On n'observe pas la formation de 1,1,1,3,3-pentachlorobutane.

### Exemples 22 à 25

On a préparé du 1,1,1,3,3-pentachlorobutane en présence de différents solvants et d'acétylacétonate de cuivre (II) à titre de catalyseur. La durée de réaction était de 2 heures. Les rapports molaires des réactifs, les températures de réaction et les résultats obtenus sont rassemblés dans le tableau V.

### Exemples 26-27

On a préparé du 1,1,1,3,3-pentachlorobutane au départ de 2-chloroprop-1-ène et de tétrachlorure de carbone en présence d'acétylacétonate de cuivre (II) à titre de catalyseur et d'un mélange de 4 trialkylphosphine oxydes (tri-(n-hexyl)phosphine oxyde, tri-(n-octyl)phosphine oxyde,
n-octyl-di-(n-hexyl)-phosphine oxyde et n-hexyl-di-(n-octyl)-phosphine oxyde), commercialisé par CYTEC sous la dénomination CYANEX® 923. La durée de réaction était de 2 heures. Les rapports molaires des réactifs, les températures de réaction et les résultats obtenus sont également présentés dans le tableau V.

**Tableau V**

| Ex. | Solvant | Rapport molaire 2-CPe/CCl₄ / Cu(acac)₂ / solvant | Température | Conversion | Sélectivité |
|---|---|---|---|---|---|
| 22 | N-méthylpyrrolidone | 1 / 2 / 0,06 / 3,8 | 100 °C | 96 | 97 |
| 23 | N,N-diméthylacétamide | 1 / 3,5 / 0,06 / 4,1 | 90 °C | 67 | 99 |
| 24 | 1,3-diméthyl-2-imidazolidinone | 1 / 2,2 / 0,05 / 3,2 | 90 °C | 46 | 97 |
| 25 | N,N-diméthylformamide | 1 / 1,9 / 0,06 / 5,7 | 100 °C | 72 | 96 |
| 26 | CYANEX® 923 | 1 / 1,8 / 0,047 / 0,99 | 90 °C | 86 | 95 |
| 27 | CYANEX® 923 | 1 / 5 / 0,05 / 0,24 | 90 °C | 88 | 95 |

### Exemple 28

On a répété l'exemple 24 avec de l'acétate de cuivre (II) comme catalyseur. On a obtenu un taux de conversion de 55 % et une sélectivité en 1,1,1,3,3-pentachlorobutane de 94 %.

### Exemple 29

On a préparé du 1,1,1,3,3-pentachlorobutane en présence de CuCl₂ comme catalyseur et de N-méthylpyrrolidone comme solvant. Le rapport molaire 2 chloroprop-1-ène/CCl₄/CuCl₂/N-méthylpyrrolidone était 1/2,2/0,05/3,3. Après 5 heures de réaction à 115 °C, le taux de conversion était de 100 % et la sélectivité en 1,1,1,3,3-pentachlorobutane de 94 %.

## Revendications (Revendications pour l'(les) Etat(s) contractant(s) suivant(s): AT, CH, LI, DK, FI, IE, LU, PT, SE)

1. Procédé de préparation de 1,1,1,3,3-pentachlorobutane par réaction entre du tétrachlorure de carbone et du 2-chloroprop-1-ène en présence d'un catalyseur de télomérisation **caractérisé en ce que** le catalyseur comprend au moins un composé de cuivre (I) ou un composé de cuivre (II).

2. Procédé selon la revendication 1 **caractérisé en ce que** le composé de cuivre est choisi parmi les halogénures de cuivre, les sels mixtes de cuivre, les carboxylates de cuivre, les complexes avec une phosphine ou avec l'acétylacétone, ou leurs mélanges.

3. Procédé selon la revendication 2 **caractérisé en ce que** le catalyseur est choisi parmi l'acétate de cuivre (II), l'hydroxychlorure de cuivre (II), le cyclohexanebutyrate de cuivre (II), l'acétylacétonate de cuivre (II) et le chlorure de cuivre (II).

4. Procédé selon l'une quelconque des revendications 1 à 3 **caractérisé en ce que** la réaction se déroule en présence d'un solvant.

5. Procédé selon la revendication 4 **caractérisé en ce que** le solvant est un nitrile, un amide ou un oxyde de trialkylphosphine.

6. Procédé selon la revendication 5 **caractérisé en ce que** le solvant est choisi parmi la N-méthylpyrrolidone, le N,N-diméthylacétamide, l'oxyde de tri-(n-hexyl)phosphine, l'oxyde de tri-(n-octyl)phosphine, l'oxyde de n-octyl-di-(n-hexyl)-phosphine, l'oxyde de n-hexyl-di-(n-octyl)-phosphine et leurs mélanges.

7. Procédé selon l'une quelconque des revendications 1 à 6 **caractérisé en ce que** la réaction se déroule en présence d'un cocatalyseur.

8. Procédé selon la revendication 7 **caractérisé en ce que** le cocatalyseur est une amine.

9. Procédé selon la revendication 8 **caractérisé en ce que** l'amine est l'isopropylamine ou la tert-butylamine.

10. Procédé selon l'une quelconque des revendications 1 à 9 **caractérisé en ce que** la réaction se déroule à une température supérieure ou égalé à 40 °C et inférieure ou égale à 200 °C.

11. Procédé d'obtention de 1,1,1,3,3-pentafluorobutane (HFC-365mfc) par fluoration de 1,1,1,3,3-pentachlorobutane obtenu selon le procédé selon l'une quelconque des revendications 1 à 11.

12. Procédé selon la revendication 11 dans lequel le 1,1,1,3,3-pentafluorobutane est obtenu par traitement avec du fluorure d'hydrogène en présence d'un catalyseur.

13. Procédé selon la revendication 12, dans lequel le catalyseur est choisi parmi les sels d'antimoine, les sels de titane et les sels d'étain.

## Revendications (Revendications pour l'(les) Etat(s) contractant(s) suivant(s): BE, DE, ES, FR, GB, GR, IT, NL)

1. Procédé de préparation de 1,1,1,3,3-pentachlorobutane par réaction entre du tétrachlorure de carbone et du 2-chloroprop-1-ène en présence d'un catalyseur de télomérisation **caractérisé en ce que** le catalyseur comprend au moins un composé de cuivre (I) ou un composé de cuivre (II), à l'exception d'une réaction en présence d'un catalyseur constitué de chlorure cuivreux avec une amine choisie parmi la n-butylamine, l'isopropylamine, la tertbutylamine, l'éthanolamine, la diéthylamine, la triéthylamine et la cyclohexylamine.

2. Procédé de préparation de 1,1,1,3,3-pentachlorobutane par réaction entre du tétrachlorure de carbone et du 2-chloroprop-1-ène en présence d'un catalyseur de télomérisation **caractérisé en ce que** le catalyseur comprend au moins un composé de cuivre (II).

3. Procédé selon la revendication 1 ou 2 **caractérisé en ce que** le composé de cuivre est choisi parmi les halogénures de cuivre (II), le bromure de cuivre (I), l'iodure de cuivre (I), les sels mixtes de cuivre, les carboxylates de cuivre, les complexes avec une phosphine ou avec l'acétylacétone, ou leurs mélanges.

4. Procédé selon la revendication 1 ou 2 **caractérisé en ce que** le catalyseur est choisi parmi l'acétate de cuivre (II), l'hydroxychlorure de cuivre (II), le cyclohexanebutyrate de cuivre (II), l'acétylacétonate de cuivre (II) et le chlorure de cuivre (II).

5. Procédé selon l'une quelconque des revendications 1 à 4 **caractérisé en ce que** la réaction se déroule en présence d'un solvant

6. Procédé selon la revendication 5 **caractérisé en ce que** le solvant est un nitrile, un amide ou un oxyde de trialkylphosphine.

7. Procédé selon la revendication 6 **caractérisé en ce que** le solvant est choisi parmi la N-méthylpyrrolidone, le N,N-diméthylacétamide, l'oxyde de tri-(n-hexyl)phosphine, l'oxyde de tri-(n-octyl)phosphine, l'oxyde de n-octyl-di-(n-hexyl)-phosphine, l'oxyde de n-hexyl-di-(n-octyl)-phosphine et leurs mélanges.

8. Procédé selon l'une quelconque des revendications 1 à 7 **caractérisé en ce que** la réaction se déroule en présence d'un cocatalyseur.

9. Procédé selon la revendication 8 **caractérisé en ce que** le cocatalyseur est une amine.

10. Procédé selon la revendication 9 **caractérisé en ce que** l'amine est l'isopropylamine ou la tert-butylamine.

11. Procédé selon l'une quelconque des revendications 1 à 10 **caractérisé en ce que** la réaction se déroule à une température supérieure ou égale à 40 °C et inférieure où égale à 200 °C.

12. Procédé d'obtention de 1,1,1,3,3-pentafluorobutane (HFC-365mfc) par fluoration de 1,1,1,3,3-pentachlorobutane obtenu selon le procédé selon l'une quelconque des revendications 1 à 11.

13. Procédé selon la revendication 12 dans lequel le 1,1,1,3,3-pentafluorobutane est obtenu par traitement avec du fluorure d'hydrogène en présence d'un catalyseur.

14. Procédé selon la revendication 13, dans lequel le catalyseur est choisi parmi les sels d'antimoine, les sels de titane, les sels de tantale et les sels d'étain.

## Patentansprüche (Patentansprüche für folgende(n) Vertragsstaat(en): AT, CH, LI, DK, FI, IE, LU, PT, SE)

1. Verfahren zur Herstellung von 1,1,1,3,3-Pentachlorbutan durch Reaktion zwischen Tetrachlorkohlenstoff und 2-Chlorprop-1-en in Gegenwart eines Telomerisationskatalysators, **dadurch gekennzeichnet, daß** der Katalysator wenigstens eine Kupfer(I)verbindung oder eine Kupfer(II)verbindung umfaßt.

2. Verfahren nach Anspruch 1, **dadurch gekennzeichnet, daß** die Kupferverbindung unter den Kupferhalogeniden, den gemischten Kupfersalzen, den Kupfercarboxylaten, den Komplexen mit einem Phosphin oder mit Acetylaceton oder unter deren Gemischen ausgewählt wird.

3. Verfahren nach Anspruch 2, **dadurch gekennzeichnet, daß** der Katalysator unter Kupfer(II)acetat, Kupfer(II)hydroxychlorid, Kupfer(II)cyclohexanbutyrat, Kupfer(II)acetylacetonat und Kupfer(II)chlorid ausgewählt wird.

4. Verfahren nach einem der Ansprüche 1 bis 3, **dadurch gekennzeichnet, daß** die Umsetzung in Gegenwart eines Lösungsmittels abläuft.

5. Verfahren nach Anspruch 4, **dadurch gekennzeichnet, daß** das Lösungsmittel ein Nitril, ein Amid oder ein Trialkylphosphinoxid ist.

6. Verfahren nach Anspruch 5, **dadurch gekennzeichnet, daß** das Lösungsmittel unter N-Methylpyrrolidon, N,N-Dimethylacetamid, Tri(n-hexyl)phosphinoxid, Tri(n-octyl)phosphinoxid, n-Octyl-di(n-hexyl)phosphinoxid, n-Hexyl-di(n-octyl)phosphinoxid und deren Gemischen ausgewählt wird.

7. Verfahren nach einem der Ansprüche 1 bis 6, **dadurch gekennzeichnet, daß** die Umsetzung in Gegenwart eines Cokatalysators abläuft.

8. Verfahren nach Anspruch 7, **dadurch gekennzeichnet, daß** der Cokatalysator ein Amin ist.

9. Verfahren nach Anspruch 8, **dadurch gekennzeichnet, daß** das Amin das Isopropylamin oder das tert.-Butylamin ist.

10. Verfahren nach einem der Ansprüche 1 bis 9, **dadurch gekennzeichnet, daß** die Umsetzung bei einer Temperatur von größer als oder gleich 40°C und kleiner als oder gleich 200°C abläuft.

11. Verfahren zur Gewinnung von 1,1,1,3,3-Pentafluorbutan(HFC-365mfc) durch Fluorierung von 1,1,1,3,3-Pentachlorbutan, das nach dem Verfahren gemäß einem der Ansprüche 1 bis 10 erhalten worden ist.

12. Verfahren nach Anspruch 11, worin das 1,1,1,3,3-Pentafluorbutan durch Behandlung mit Fluorwasserstoff in Gegenwart eines Katalysators erhalten wird.

13. Verfahren nach Anspruch 12, worin der Katalysator unter Antimonsalzen, Titansalzen und Zinnsalzen ausgewählt wird.

## Patentansprüche (Patentansprüche für folgende(n) Vertragsstaat(en): BE, DE, ES, FR, GB, GR, IT, NL)

1. Verfahren zur Herstellung von 1,1,1,3,3-Pentachlorbutan durch Reaktion zwischen Tetrachlorkohlenstoff und 2-Chlorprop-1-en in Gegenwart eines Telomerisationskatalysators, **dadurch gekennzeichnet, daß** der Katalysator wenigstens eine Kupfer(I)verbindung oder eine Kupfer(II)verbindung umfaßt, mit Ausnahme einer Reaktion in Gegenwart eines Katalysators, der aus Kupfer(I)chlorid mit einem unter n-Butylamin, Isopropylamin, tert.-Butylamin, Ethanolamin, Diethylamin, Triethylamin und Cyclohexylamin ausgewählten Amin gebildet ist.

2. Verfahren zur Herstellung von 1,1,1,3,3-Pentachlorbutan durch Reaktion zwischen Tetrachlorkohlenstoff und 2-Chlorprop-1-en in Gegenwart eines Telomerisationskatalysators, **dadurch gekennzeichnet, daß** der Katalysator wenigstens eine Kupfer(II)verbindung umfaßt.

3. Verfahren nach Anspruch 1 oder 2, **dadurch gekennzeichnet, daß** die Kupferverbindung unter den Kupfer(II)halogeniden, dem Kupfer(I)bromid, dem Kupfer(I)jodid, den gemischten Kupfersalzen, den Kupfercarboxylaten, den Komplexen mit einem Phosphin oder mit Acetylaceton oder unter deren Gemischen ausgewählt wird.

4. Verfahren nach Anspruch 1 oder 2, **dadurch gekennzeichnet, daß** der Katalysator unter Kupfer(II)acetat, Kupfer(II)-hydroxychlorid, Kupfer(II)cyclohexanbutyrat, Kupfer(II)-acetylacetonat und Kupfer(II)chlorid ausgewählt wird.

5. Verfahren nach einem der Ansprüche 1 bis 4, **dadurch gekennzeichnet, daß** die Umsetzung in Gegenwart eines Lösungsmittels abläuft.

6. Verfahren nach Anspruch 5, **dadurch gekennzeichnet, daß** das Lösungsmittel ein Nitril, ein Amid oder ein Trialkylphosphinoxid ist.

7. Verfahren nach Anspruch 6, **dadurch gekennzeichnet, daß** das Lösungsmittel unter N-Methylpyrrolidon, N,N-Dimethylacetamid, Tri(n-hexyl)phosphinoxid, Tri(n-octyl)phosphinoxid, n-Octyl-di(n-hexyl)phosphinoxid, n-Hexyl-di(n-octyl)phosphinoxid und deren Gemischen ausgewählt wird.

8. Verfahren nach einem der Ansprüche 1 bis 7, **dadurch gekennzeichnet, daß** die Umsetzung in Gegenwart eines Cokatalysators abläuft.

9. Verfahren nach Anspruch 8, **dadurch gekennzeichnet, daß** der Cokatalysator ein Amin ist.

10. Verfahren nach Anspruch 9, **dadurch gekennzeichnet, daß** das Amin das Isopropylamin oder das tert.-Butylamin ist.

11. Verfahren nach einem der Ansprüche 1 bis 10, **dadurch gekennzeichnet, daß** die Umsetzung bei einer Temperatur von größer als oder gleich 40°C und kleiner als oder gleich 200°C abläuft.

12. Verfahren zur Gewinnung von 1,1,1,3,3-Pentafluorbutan(HFC-365mfc) durch Fluorierung von 1,1,1,3,3-Pentachlorbutan, das nach dem Verfahren gemäß einem der Ansprüche 1 bis 11 erhalten worden ist.

13. Verfahren nach Anspruch 12, worin das 1,1,1,3,3-Pentafluorbutan durch Behandlung mit Fluorwasserstoff in Gegenwart eines Katalysators erhalten wird.

14. Verfahren nach Anspruch 13, worin der Katalysator unter Antimonsalzen, Titansalzen, Tantalsalzen und Zinnsalzen ausgewählt wird.

## Claims (Claims for the following Contracting State(s): AT, CH, LI, DK, FI, IE, LU, PT, SE)

1. Process for the preparation of 1,1,1,3,3-pentachlorobutane by reacting carbon tetrachloride with 2-chloro-1-propene in the presence of a telomerization catalyst, **characterized in that** the catalyst comprises at least one copper (I) compound or at least one copper (II) compound.

2. Process according to Claim 1, **characterized in that** the copper compound is chosen from copper halides, mixed copper salts, copper carboxylates and complexes with a phosphine or with acetylacetone, or mixtures thereof.

3. Process according to Claim 2, **characterized in that** the catalyst is chosen from copper (II) acetate, copper (II) hydroxychloride, copper (II) cyclohexanebutyrate, copper (II) acetylacetonate and copper (II) chloride.

4. Process according to any one of Claims 1 to 3, **characterized in that** the reaction takes place in the presence of a solvent.

5. Process according to Claim 4, **characterized in that** the solvent is a nitrile, an amide or a trialkylphosphine oxide.

6. Process according to Claim 5, **characterized in that** the solvent is chosen from N-methylpyrrolidone, N,N-dimethylacetamide, tri(n-hexyl)phosphine oxide, tri(n-octyl)phosphine oxide, n-octyldi(n-hexyl)phosphine oxide and n-hexyldi(n-octyl)phosphine oxide, and mixtures thereof.

7. Process according to any one of Claims 1 to 6, **characterized in that** the reaction takes place in the presence of a cocatalyst.

8. Process according to Claim 7, **characterized in that** the cocatalyst is an amine.

9. Process according to Claim 8, **characterized in that** the amine is isopropylamine or tert-butylamine.

10. Process according to any one of Claims 1 to 9, **characterized in that** the reaction takes place at a temperature greater than or equal to 40°C and less than or equal to 200°C.

11. Process for obtaining 1,1,1,3,3-pentafluorobutane (HFC-365 mfc) by fluorination of 1,1,1,3,3-pentachlorobutane obtained by the process according to any one of Claims 1 to 10.

12. Process according to Claim 11, wherein the 1,1,1,3,3-pentafluorobutane is obtained by a treatment with hydrogen fluoride in the presence of a catalyst.

13. Process according to Claim 12, wherein the catalyst is chosen from antimony salts, titanium salts and tin salts.

## Claims (Claims for the following Contracting State(s): BE, DE, ES, FR, GB, GR, IT, NL)

1. Process for the preparation of 1,1,1,3,3-pentachlorobutane by reacting carbon tetrachloride with 2-chloro-1-propene in the presence of a telomerization catalyst, **characterized in that** the catalyst comprises at least one copper (I) compound or at least one copper (II) compound, with the proviso that a reaction carried out in the presence of a catalyst consisting of cuprous chloride and of an amine chosen from n-butylamine, isopropylamine, tertbutylamine, ethanolamine, diethylamine, trietylamine and cycloethylamine is excluded.

2. Process for the preparation of 1,1,1,3,3pentachlorobutane by reacting carbon tetrachloride with 2-chloro-1-propene in the presence of a telomerisation catalyst **characterized in that** the catalyst comprises at least one copper (II) compound.

3. Process according to Claim 1 or 2, **characterized in that** the copper compound is chosen from copper (II) halides, copper (I) bromide, copper (I) iodide, mixed copper salts, copper carboxylates and complexes with a phosphine or with acetylacetone, or mixtures thereof.

4. Process according to Claim 1 or 2, **characterized in that** the catalyst is chosen from copper (II) acetate, copper (II) hydroxychloride, copper (II) cyclohexanebutyrate, copper (II) acetylacetonate and copper (II) chloride.

5. Process according to any one of Claims 1 to 4, **characterized in that** the reaction takes place in the presence of a solvent.

6. Process according to Claim 5, **characterized in that** the solvent is a nitrile, an amide or a trialkylphosphine oxide.

7. Process according to Claim 6, **characterized in that** the solvent is chosen from N-methylpyrrolidone, N,N-dimethylacetamide, tri(n-hexyl)phosphine oxide, tri(n-octyl)phosphine oxide, n-octyldi(n-hexyl)phosphine oxide and n-hexyldi(n-octyl)phosphine oxide, and mixtures thereof.

8. Process according to any one of Claims 1 to 7, **characterized in that** the reaction takes place in the presence of a cocatalyst.

9. Process according to Claim 8, **characterized in that** the cocatalyst is an amine.

10. Process according to Claim 9, **characterized in that** the amine is isopropylamine or tert-butylamine.

11. Process according to any one of Claims 1 to 9, **characterized in that** the reaction takes place at a temperature greater than or equal to 40°C and less than or equal to 200°C.

12. Process for obtaining 1,1,1,3,3-pentafluorobutane (HPC-365mfc) by fluorination of 1,1,1,3,3-pentachlorobutane obtained by the process according to any one of Claims 1 to 11.

13. Process according to Claim 12, wherein the 1,1,1,3,3-pentafluorobutane is obtained by a treatment with hydrogen fluoride in the presence of a catalyst.

14. Process according to Claim 13, wherein the catalyst is chosen from antimony salts, titanium salts, tantalum salts and tin salts.
